# EUROPÄISCHE PATENTSCHRIFT

(11) **EP 4 384 126 B1**
(45) Veröffentlichungstag und Bekanntmachung des Hinweises auf die Patenterteilung: **23.04.2025**
(21) Anmeldenummer: 23757898.4
(22) Anmeldetag: 16.08.2023
(51) Int. Cl.: A61F 5/41

(54) **VORRICHTUNG ZUR DAUERHAFTEN EXTENSION EINES PENIS**
APPARATUS FOR PERMANENTLY EXTENDING A PENIS
DISPOSITIF D'EXTENSION PERMANENTE DU PÉNIS

(30) Priorität: 17.08.2022 DE 102022120804
(43) Veröffentlichungstag der Anmeldung: 19.06.2024
(73) Patentinhaber: Swiss-Tec Global Limited, 9490 Vaduz (LI)
(72) Erfinder: JOCHUM, Julian, 82343 Pöcking (DE)
(74) Vertreter: Röthinger, Rainer
(86) Internationale Anmeldenummer: PCT/EP2023/072584
(87) Internationale Veröffentlichungsnummer: WO 2024/038097

(56) Entgegenhaltungen:
- DE-U1- 20 203 927
- DE-U1- 29 521 655
- US-A1- 2004 215 055
- US-B2- 8 900 121

## Beschreibung

Die Erfindung betrifft eine Vorrichtung zur dauerhaften Extension eines Penis, die einen Stützring und mindestens zwei an dem Stützring gelenkig befestigte, mit Hilfe einer Feder axial federnd gelagerte und längenverstellbare Streckstangen umfasst , wobei die Streckstangen
eine Gelenkhülse, die an einem Ende gelenkig mit dem Stützring verbunden ist,
eine Federhülse, in der die Feder angeordnet ist und die an einem Ende mit einem Kopplungselement, das geeignet ist, eine Befestigungseinrichtung für einen Penis mit den Streckstangen zu verbinden und eine Zugkraft auf den Penis auszuüben, verbunden ist, und
mindestens eine zwischen Federhülse und Gelenkhülse angeordnete stangenförmige teleskopische Verstelleinheit
   umfassen.

Vorrichtungen in der hier in Rede stehenden Art, die auch als Penisextensionsvorrichtungen bezeichnet werden, sind in vielfältigen Ausführungsformen bekannt. So ist beispielsweise in der EP 3463218 B1 eine Befestigungsvorrichtung einer derartigen Penisextensionsvorrichtung mit einem Zugkraftmesser beschrieben. In dieser Druckschrift sind auch zahlreiche einschlägige Vorrichtungen beschrieben und gewürdigt. Auf diese Ausführungen wird hiermit Bezug genommen.

Aus der US 2004/0215055 A1 ist eine Vorrichtung zur Ausübung eines Zugs auf einen Penis bekannt, die ein ringförmiges Basiselement und zwei gelenkig an dem Basiselement befestigte Verbindungselemente aufweist. Diese Verbindungselemente der bekannten Zugvorrichtung sind mit einer Gewindestange versehen, die in einen Kolben einschraubbar ist. Das Ende des Kolbens kommt in Anlage an eine in einer Federhülse gelagerten Feder, durch welche eine Zugkraft in Längsrichtung der Streckstange ausgeübt werden kann.

Zur Längeneinstellung der Streckstangen bei dieser bekannten Zugvorrichtung dienen mehrere stabförmige Verlängerungselemente, die an einem ihrer Ende ein Gewindestift und an ihrem anderen Ende eine Gewindebohrung aufweisen. Um diese Verlängerungselemente miteinander zu verbinden, wird ein Verlängerungselement mit seinem Gewindestift in die Gewindebohrung des benachbarten Verlängerungselements eingeschraubt. An einer Kombination von zwei auf diese Weise verbundenen Verlängerungselementen kann ein drittes Verlängerungselement auf die beschriebene Weise hinzugefügt werden.

Die Gesamtlänge der endgültigen Gewindestange wird durch die Anzahl und die Länge der einzelnen auf diese Weise miteinander verbundenen Verlängerungselementen bestimmt. Die Einheit aus mehreren miteinander verbundenen Verlängerungselementen wird an einem Ende mit der Federhülse und am anderen Ende mit einer Befestigungseinrichtung für den Penis verbunden.

Die Verlängerungselemente werden mit ihren Gewindestiften vollständig in die damit korrespondierenden Gewindebohrungen eingeschraubt, damit die erforderliche Stabilität der aus mehreren Verlängerungselementen zusammengesetzten Einheit gewährleistet wird. Eine Längenverstellung durch verdrehen von Gewindebohrung gegenüber Gewindestift ist nicht vorgesehen. Eine Verlängerung oder Verkürzung der Streckstangen kann somit nur durch Hinzufügen oder Entfernen von Verlängerungselementen und somit beispielsweise nicht während des Tragens dieser bekannten Streckvorrichtung durch den Benutzer erfolgen.

In der DE 202 03 927 U1 ist eine spezielle Ausführungsform einer gattungsgemäßen Penisextensionsvorrichtung beschrieben, die dem Oberbegriff des Anspruchs 1 entspricht.

Ausgehend von letzterem Stand der Technik ist es Aufgabe der vorliegenden Erfindung, eine Vorrichtung der hier in Rede stehenden Art bereitzustellen, die einfach und flexibel an die Längenanforderungen angepasst werden kann.

Gelöst für diese Aufgabe durch eine Vorrichtung, die dadurch gekennzeichnet ist, dass die Gelenkhülse einen Innenbohrung aufweist, in deren Wandung ein Innengewinde ausgebildet ist,
zwischen Federhülse und Gelenkhülse ein erste und eine zweite teleskopische Verstelleinheit angeordnet sind,
die erste Verstelleinheit eine Gewindehülse darstellt, die ein Außengewinde, das in das Innengewinde der Gelenkhülse eingeschraubt ist, und eine Innenbohrung, in deren Wandung ein Innengewinde ausgebildet ist, besitzt,
die zweite Verstelleinheit eine Gewindespindel darstellt, die zur Gewindehülse hin mit einem Außengewinde, das in das Innengewinde der Gewindehülse eingeschraubt ist, und zur Federhülse hin mit einem in die Federhülse ragenden Steckstift ausgestattet ist, und
die Feder in der Federhülse zwischen Steckstift und Kopplungselement angeordnet ist.

Vorzugsweise ist zwischen dem Außengewinde und dem Steckstift der Gewindespindel ein radial außen umlaufender Anschlagflansch ausgebildet.

Weiterhin bevorzugt besitzt die Gewindehülse ebenfalls an ihrem distalen Ende einen radial außen umlaufenden Anschlagflansch.

Die Länge von Gewindespindel, Gewindehülse und Gelenkhüse sind zweckmäßigerweise darart gewählt und aufeinander abgestimmt, dass der gewünschte und/oder erforderliche Verstellbereich abgedeckt ist.

Das Innengewinde der Gelenkhülse ist in der Wandung der Innenbohrung der Gelenkhülse ausgebildet. Die Innenbohrung ist distal offen, proximal zweckmäßigerweise geschlossen, kann jedoch auch offen sein.

Vorzugsweise macht die axiale Länge der Innenbohrung der Gelenkhülse mindestens 80 % der axialen Länge der Gelenkhülse aus.

Das Innengewinde kann sich auf die gesamten axialen Läge des Innenbohrung oder nur über einen Abschnitt bzw. Bereich davon erstrecken.

Auch die Gewindehülse besitzt eine Innenbohrung, die distal offen ist und proximal verschlossen oder offen sein kann. Das Innengewinde der Gewindehülse ist in der Wandung der Innenbohrung ausgebildet und kann sich auf die gesamten axialen Läge der Innenbohrung oder nur über einen Abschnitt bzw. Bereich der Innenbohrung erstrecken.

Vorzugsweise macht die axiale Länge der Innenbohrung der Gewindehülse mindestens 80 % der axialen Länge der Gewindehülse aus.

Die Gewindehülse besitzt zudem ein Außengewinde, das auf der Mantelfläche der Gewindehülse ausgebildet ist und das sich über die gesamte axiale Länge der Gewindehülse oder nur einen Bereich oder Abschnitt davon erstrecken kann.

Vorzugsweise beträgt die axiale Länge, die für die Ausbildung des Außengewindes der Gewindehülse zur Verfügung steht, mindestens 80 % der gesamten axialen Länge der Gewindehülse.

Vorzugsweise beträgt die axiale Länge der Mantelfläche der Gewindehülse, auf der das Außengewindes ausgebildet sein kann, mindestens 80 % der axialen Länge der Innenbohrung der Gelenkhülse.

Weiterhin bevorzugt beträgt die axiale Länge, die für die Ausbildung des Außengewindes der Gewindespindel zur Verfügung steht, mindestens 80 % der axialen Länge der Innenbohrung der Gewindehülse.

Das Bereich der Gewindespindel, auf der das Außengewinde der Gewindespindel ausgebildet sein kann, erstreckt sich vom Steckstift bis zum proximalen Ende oder über einen Abschnitt bzw. Bereich davon.

Oben beschriebene bevorzugte Merkmale sollen sicherstellen, dass die beiden Verstelleinheiten, nämlich Gewindehülse und Gewindespindel, und die Gelenkhülse vorzugsweise so weit wie möglich ineinander geschraubt und auch wieder herausgeschraubt werden können, so dass im eingeschraubten Zustand die Gewindehülse mindestens zu 80% und sogar vollständig in die Gelenkhülse und die Gewindespindel mindestens zu 80% und sogar vollständig in die Gewindehülse eingeschraubt sind.

Sind bevorzugte Anschlagflansche vorhanden, dann sind diese natürlich nicht irgendwo eingeschraubt.

Im herausgeschraubten Zustand ist die Länge der Einheit aus Gelenkhülse und den beiden Verstelleinheiten so groß wie möglich. Insgesamt ergibt sich daher ein sehr großer Verstellbereich.

Der Begriff "heausgeschraubter Zustand" bezeichnet eine Situation, in der die miteinander verschraubten Elemente noch miteinander verbunden und somit nicht voneinander getrennt sind, jedoch so weit wie möglich in Längsrichtung voneinander entfernt sind.

Die Federhülse ist vorzugsweise zum Kopplungselement hin durch einen einrastbaren Deckel verschlossen und die Feder ist zwischen dem Deckel und dem Steckstift angeordnet.

Besonders bevorzugt ist eine Vorrichtung, die dadurch gekennzeichnet ist, dass die Streckstangen an dem Stützring derart befestigt sind, dass der Stützring pro Streckstange zwei parallele Backen besitzt, die jeweils eine nach innen offene Ausnehmung besitzen, dass die Gelenkhülsen ein Gelenkstück, das ein ringscheibenförmiges Mittelteil aufweist, an dem beidseitig jeweils eine halbkugelförmige Ausbuchtung ausgebildet sind, besitzen, die Dicke des ringscheibenfömigen Mittelteils geringer als der Abstand der Backen und die Ausbuchtungen in die gegenüberliegende Ausnehmung eingreifen.

Das Kopplungselement der erfindungsgemäßen Vorrichtung ist vorzugsweise mit einer Befestigungseinrichtung für einen Penis verbunden. Diese Befestigungseinrichtung ist bekannter Natur und beispielsweise beschrieben in EP 3 463 218 B1.

Nach einer alternativen Ausführungsform kann das Kopplungselement als solches als eine Befestigungseinrichtung für einen Penis ausgestaltet sein. Auch eine derartige Befestigungseinrichtung ist bekannt, beispielsweise aus der DE 200 03 927 U1.

Der hier verwendete Ausdruck "Feder" steht für ein elastisches Element, das beim Komprimieren eine Rückstellkraft in die Ausgangslage entwickelt. Die Feder der erfindungsgemäßen Vorrichtung stellt vorzugsweise eine Druckfeder bzw. eine Helixfeder aus insbesondere einem Metall, ein Stäbchen oder ein Röhrchen aus einem elastischen Material dar.

Als elastisches Material kann ein Schaummaterial und vorzugsweise Silicon dienen.

Bein den weiteren Elementen der erfindungsgemäßen Vorrichtung handelt es sich insbesondere um Spritzgussteile aus einem bekannten, dafür geeigneten Kunststoffmaterial.

Bei diesem Kunststoffmaterial kann es sich um ein durchsichtiges bzw. transparentes Material handeln. Dies gilt insbesondere für die Gelenkhülse und die Gewindehülse, da es auf diese Weise möglich ist, visuell festzustellen, wie weit die Gewindehülse in die Gelenkhülse bzw. die Gewindespindel in die Gewindehülse eingedreht sind.

Die Erfindung wird im Folgenden anhand der nicht maßstabsgetreuen, beispielhaften Zeichnungen näher beschrieben. Von den Figuren zeigen:
Figur 1 eine Aufsicht von schräg oben auf eine erfindungsgemäße Vorrichtung, teilweise in Explosionsdarstellung,
Figur 2 eine der Figur 1 entsprechende Ansicht, jedoch als vollständige Explosionsdarstellung,
Figur 3 eine Aufsicht von oben der in der Figur 1 gezeigten Vorrichtung im zusammengebauten Zustand, bei der die Gewindehülse und die beiden Verstelleinheiten ineinander geschraubt sind,
Figur 4 eine Seitenansicht der in der Figur 3 gezeigten Vorrichtung,
Figur 5 zwei Seitenansichten einer Gelenkshülse, in die eine erste Verstelleinheit vollständig eingeschraubt ist, wobei in die erste Verstelleinheit eine zweite Verstelleinheit vollständig eingeschraubt ist sowie einen Längsschnitt entlang A-A,
Figur 6 eine Seitenansicht der ersten Versstelleinheit in Form einer Gewindehülse sowie einen Längsschnitt entlang A-A,
Figur 7 eine Seitenansicht einer zweiten Versstelleinheit in Form einer Gewindespindel sowie einen Längsschnitt entlang A-A,
Figur 8 eine Seitenansicht einer Federhülse sowie einen Längsschnitt entlang A-A,
Figur 9 eine Seitenansicht eines Deckels für die Federhülse sowie einen Längsschnitt entlang A-A und
Figur 10 eine perspektivische Ansicht einer Befestigungseinrichtung zum Verbinden eines Penis mit einer Penisextensionsvorrichtung, wobei diese Befestigungseinrichtung unter anderem aus der EP 3 463 218 B1 bekannt ist.

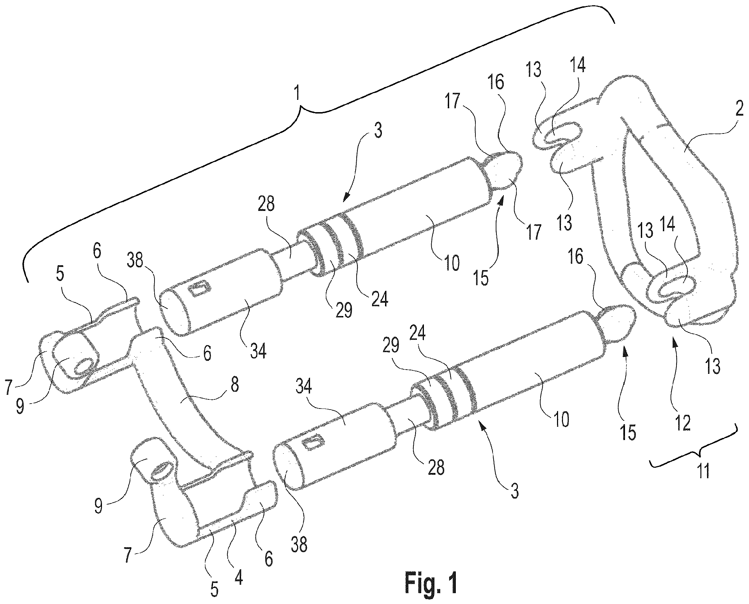

Die in der Figur 1 in einer Ansicht von schräg oben gezeigte, erfindungsgemäße Vorrichtung 1 weist drei Bestandteile auf, nämlich einen Stützring 2, zwei längenverstellbare Streckstangen 3 und ein Kopplungselement 4 auf. Diese drei Bestandteile sind separat und daher im nicht miteinander verbundenen Zustand gezeigt.

Der Stützring 2 dient zur Abstützung der Vorrichtung 1 am Körper des Benutzers und stellt den proximalen Teil der Vorrichtung 1 dar. Ein derartiger Stützring 2 ist aus dem eingangs beschriebenen Stand der Technik bekannt.

Das distale, dem Stützring 2 gegenüberliegende Ende der Streckstangen 3 ist in einem distal durch einen Deckel 7 verschlossenen zylindrischen, seitlich in Längsrichtung offenen hohlzylindrischen Aufnahmeteil 5 des Kopplungselements 4 angeordnet. Das Aufnahmeteil 5 weist federnde Wangen 6 auf, auf die nachstehend noch näher eingegangen wird. Zur Verbindung der beiden Aufnahmeteile 5 dient ein Verbindungselement 8, das an seinen beiden Enden an der zugehörigen Wange 6 befestigt ist. An dem Deckel 7 ist eine Lagerbuchse 9 befestigt. Die oben beschriebenen Elemente des Kopplungselements 4 sind insbesondere einstückig spritzgeformt.

An dem Stützring 2 sind zwei schwenkbar am Stützring 2 angelenkte, aus mehreren Elementen aufgebauten Streckstangen 3 angebracht.

Am proximalen Ende besitzt die Streckstange 3 eine Gelenkhülse 10, die an ihrem proximalen Ende am Stützring 2 mittels eines Gelenks 11 schwenkbar sowie mit seitlichem Spiel angelenkt ist.

Das Gelenk 11 besitzt eine Gelenkaufnahme 12, die am Stützring 2 befestigt ist. Zu jeder Gelenkaufnahme 12 gehören zwei parallel zueinander ausgerichtete Backen 13, die jeweils eine nach innen offene und somit zur gegenüberliegenden Backe 13 offene Ausnehmung 14 besitzen. Stützring 2 zusammen mit Backen 13 sind einstückig spritzgeformt.

Zum Gelenk 11 gehört ferner ein Gelenkstück 15 an der Gelenkhülse 10, das ein ringsscheibenförmiges Mittelteil 16 besitzt, an dem beidseitig jeweils eine halbkugelförmige Ausbuchtung 17 ausgebildet ist.

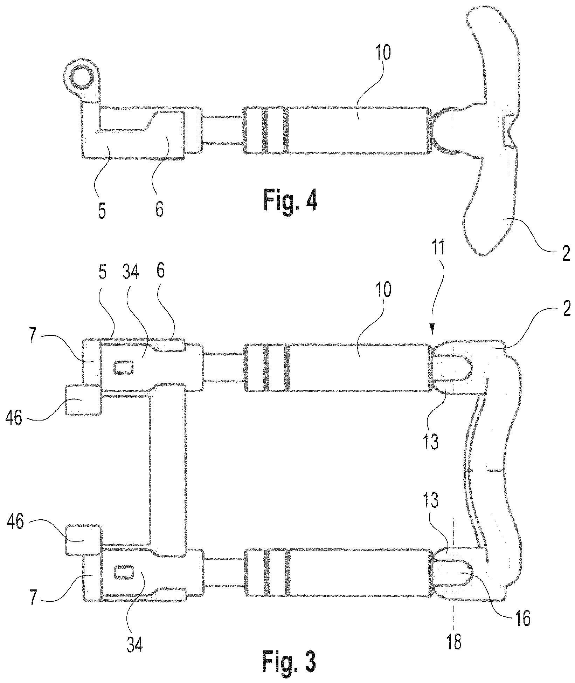

Wie insbesondere aus der Figur 3 ersichtlich ist, kommt beim zusammengebauten Gelenk 11 das Mittelteil 16 zwischen den beiden einander gegenüberliegenden Backen 13 der Gelenkaufnahme 12 zu liegen, wobei die halbkugelförmigen Ausbuchtungen 17 in der gegenüberliegende Ausnehmung 14 der Backe 13 angeordnet sind. Die Dicke des Mittelteils 16 ist dabei geringer als der Abstand der beiden Backen 13. Dies hat zur Folge, dass das Gelenkhülse 10 und somit die Streckstangen 2 nicht nur eine Drehbewegung um die durch die beiden Ausnehmungen 14 verlaufende Achse 18 vollziehen kann, sondern auch ein Schwenken in Richtung dieser Achse 18 ermöglicht wird.

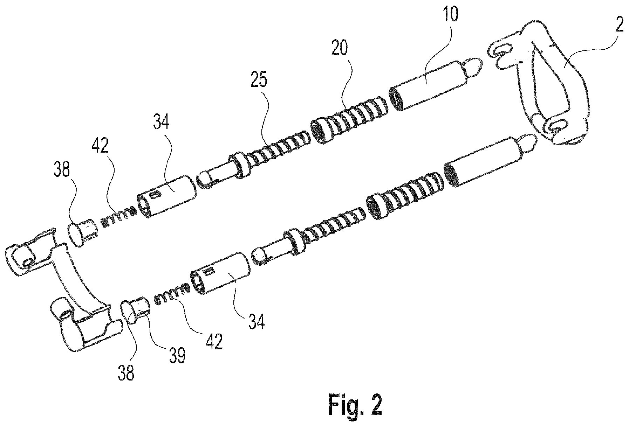

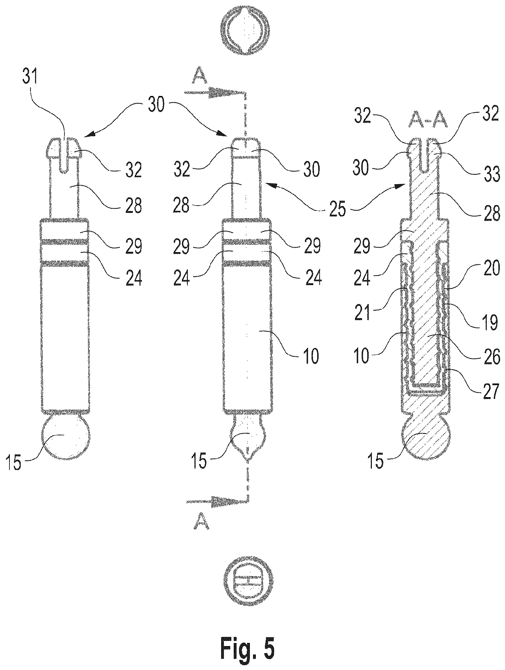

Wie insbesondere aus den Figuren 2 und 5 ersichtlich ist, besitzt die Gelenkhülse 10 eine sich in Längsrichtung erstreckende, mit einem Gewinde ausgestattete Innenbohrung 19, die zu ihrem distalen Ende offen ist.

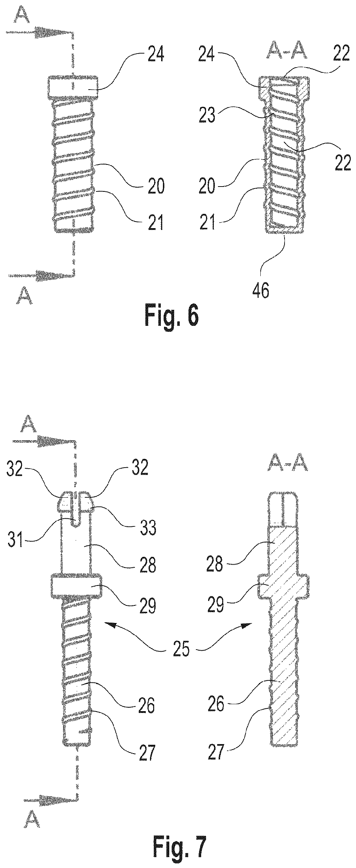

In die Innenbohrung 19 der Gelenkhülse 10 ist eine in der Figur 6 näher gezeigte erste teleskopische Verstelleinheit in Form einer hohlzylindrischen Gewindehülse 20 eingeschraubt. Die Gewindehülse 20 ist dazu auf ihrer Außenmantelfläche mit einem Außengewinde 21 ausgestattet, das derart dimensionierte und geformt ist, dass es in das Gewinde der Innenbohrung 19 der Gelenkhülse 10 einschraubbar ist. Die Innenbohrung 19 ist proximal durch eine Wand 46 verschlossen und stellt eine Art Sackbohrung dar.

Die Gewindehülse 20 besitzt ferner eine sich in Längsrichtung erstreckende zylindrische, mit einem Gewinde 23 versehene Innenbohrung 22 sowie an ihrem distalen Ende einen radial umlaufenden, sich nach radial außen erstreckenden Anschlagflansch 24.

An die Gewindehülse 20 schließt sich distal eine zweite teleskopische Verstelleinheit in Form einer Gewindespindel 25 (man vergleiche insbesondere Figur 7) an. Diese Gewindespindel 25 besitzt zwei Abschnitte, nämlich einen proximalen Abschnitt in Form eines Zylinderstiftes 26 mit einem Außengewinde 27 und einen distalen Abschnitt in Form eines zylindrischen Steckstiftes 28. Zwischen diesen beiden Abschnitten ist ein radial umlaufender, sich nach radial außen erstreckender Anschlagflansch 29 angeordnet.

Das Außengewinde 27 der Gewindespindel 25 ist derart bemessen und geformt, dass die Gewindespindel 25 in die Gewindehülse 20 einschraubbar ist, bis der Anschlagflansch 29 der Gewindespindel 25 gegen den Anschlagflansch 24 der Gewindehülse 20 in Anschlag kommt. Die beiden Anschlagflansche 24 und 29 besitzen dabei die gleichen Außendurchmesser sowie die gleiche axiale Dicke. Dieser Außendurchmesser entspricht dem Außendurchmesser der Gelenkhülse 10.

Am distalen Ende der Gewindespindel 25 ist ein Rastspitze 30 angeordnet, die einen in Längsrichtung verlaufenden Einschnitt 31 sitzt, wodurch zwei einander gegenüberliegende Rastzungen 32 gebildet werden. Am Übergang von Rastzunge 32 zum Steckstift 28 ist eine nach radial außen zeigende sowie radial umlaufende Rastnase 33 ausgebildet.

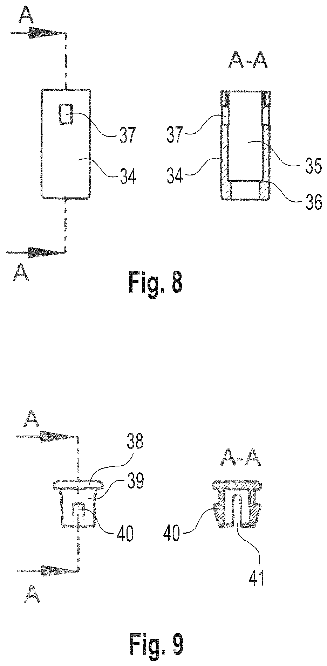

An die Gewindespindel 25 schließt sich distal eine außenzylindrische Federhülse 34 (man vergleiche insbesondere Figur 8) an, die über eine durchgehende in Längsrichtung angeordnete zylindrische Innenbohrung 35 verfügt. Der Durchmesser dieser Innenbohrung 35 erweitert sich in distaler Richtung in Form eines Absatzes 36. Beim Einführen der Rastspitze 30 in das proximale Ende der Federhülse 34 werden die beiden Rastzungen 32 zusammengedrückt, bis sie in den Bereich mit dem größeren Durchmesser der Innenbohrung 35 gelangen und sich wieder voneinander entfernen. Durch das aufliegen der Rastnasen 33 auf dem Absatz 30 wird ein Herausziehen der Rastspitze 30 und somit der Gewindespindel 25 verhindert.

Die Federhülse 34 besitzt zwei diametral gegenüberliegende Bohrungen 37 mit in Aufsicht in etwa rechteckiger Form, die sich durch die Wand der Federhülse 34 erstrecken. Zum Verschluss des distalen Endes der Federhülse 34 dient ein in der Figur 9 genauer gezeigte Deckel 38, der einen hohlzylindrischen Fortsatz 39 besitzt, welcher derart dimensioniert und geformt ist, dass er in das distale Ende der Federhülse 34 einsetzbar ist. Das freie Ende dieses Fortsatzes 39 ist mit einem Einschnitt 41 versehen und besitzt zwei Rastnasen 40, die in die dazugehörige Bohrung 37 in der Federhülse 34 eingreifen.

In die Federhülse 34 ist eine Druckfeder 42 eingesetzt, die sich zwischen dem Deckel 38 und der Rastspitze 30 befindet.

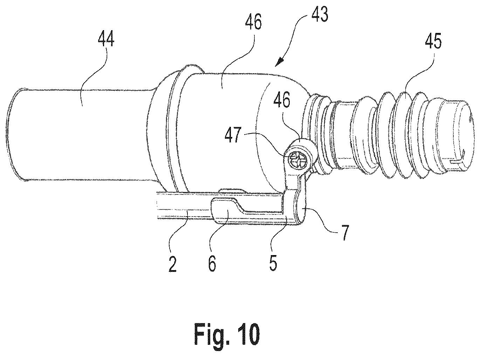

Das Kopplungselement 4 dient bei einer Ausführungsform dazu, eine per se bekannte Verbindungsvorrichtung 43 mit dem Kopplungselement 4 zu verbinden. In der Figur 10 ist ein derartige, unter anderem aus EP 3 463 218 B1 bekannte Befestigungseinrichtung 43 bekannt, die eine flexible Manschette 44 und einen Hohlkörper 46 zur Aufnahme eines Penis aufweist. Nach einführen des Penis in den Hohlkörper 46 wird die Manschette 44 auf dem Penis angebracht, beispielsweise durch Abrollen der zuvor auf den Hohlkörper 46 aufgerollten Manschette 44 auf den Penis. Durch die Vakuumpumpe 45 kann ein Unterdruck erzeugt werden, sodass eine stabile Verbindung zwischen Penis und Befestigungseinrichtung 43 erzeugt wird. All dies ist bekannt.

Die Befestigungseinrichtung 43 ist mit dem Kopplungselemente 4 der erfindungsgemäßen Vorrichtung 1 über das hohlzylindrische Aufnahmeteil 5, das mit einem Deckel 7 ausgestattet ist, verbunden. An diesem Deckel 7 ist eine Lagerbuchse 9 (siehe auch Figur 1) befestigt bzw. angeformt, in der ein Lagerzapfen 47 drehbar gelagert ist, welcher mit der der Befestigungseinrichtung 43 verbunden ist.

Zum Anbringen der erfindungsgemäßen Vorrichtung 1 kann man wie folgt vorgehen. Jede Streckstange 3 wird in dem dazugehörigen Gelenk 11 gelagert, indem das Gelenksstück 15 zwischen die Backen 13 unter Aufweitung davon eingeschoben werden. Die Gewindehülse 20 wird in die Gelenkhülse 10 ebenfalls eingedreht bzw. eingeschraubt. Zudem wird die Gewindespindel 25 mit ihrem Zylinderstift 26 in die Gewindehülse 20 eingeschraubt bzw. eingedreht. Der Steckstift 28 der Gewindespindel 25 wird in die Federhülse 34 eingesteckt, bis die Rastspitze 30 im Inneren der Federhülse 34 einrastet. In die Federhülse 34 wird die Druckfeder 42 eingesetzt. Daraufhin wird die Innenbohrung 35 der Federhülse 34 durch den Deckel 38, der mit seinem Rastnasen 40 in die Bohrungen 37 einrastet, verschlossen. Der Deckel 38 ist von der Rastnase 33 beanstandet. In diesem Raum befindet sich die Druckfeder 48, die bei komprimiert wird, wenn die der Steckstift 28 in die Federhülse 34 ein geschoben wird.

Die Federhülse 34 wird in das dazugehörigen hohlzylindrischen Aufnahmeteile 5 eingeschoben oder unter Aufweitung der federnden Wangen 6 eingeclipst. Im zusammengebauten Zustand liegt der Deckel 38 an dem axial gegenüberliegenden Deckel 7 des Aufnahmeteil 25 innen an. Dieser zusammen gebaute Zustand der erfindungsgemäßen Vorrichtung 1 ist unter anderem in den Figuren 3 und 4 gezeigt. Diese Vorgänge werden für jede Streckstange 3 durchgeführt.

Die an der Verbindungsvorrichtung 43 angebrachten Lagerzapfen 47 werden in die an den Deckeln 7 angebrachten Lagerbuchsen 9 eingesetzt bzw. eingeschoben. Dieser Zustand ist in der Figur 10 dargestellt.

Es ist natürlich auch möglich, die Befestigungseinrichtung 43 zuerst mit dem Kopplungselement 4 auf die oben beschriebene Weise zu verbinden und danach die Federhülse 34 in die Aufnahmeteile 5 einzusetzen.

Der Penis kann vor oder nach dem Zusammenbau der erfindungsgemäßen Vorrichtung in die flexible Manschette 44, die eine Art Kondom darstellt, und den Hohlkörper 48 eingeführt werden.

Es ist übrigens auch möglich, statt der oben beschriebenen Befestigungseinrichtung 43 das aus der DE 202 03 927 U1 bekannte Befestigungsmittel einzusetzen, um den Penis mit der Vorrichtung 1 zu verbinden. In diesem Fall stellt das Kopplungselement 4 ein Befestigungsmittel gemäß der DE 202 03 927 U1 dar.

Um auf den Penis eine Zugkraft auszuüben, werden entweder die Gelenkhülse 10 oder die Gewindespindel 25 oder sowohl die Gelenkhülse 10 als auch die Gewindespindel 25 aus ihrer weitesten eingeschraubten Position (man vergleiche Figuren 3 und 4) herausgeschraubt. Dabei verlängert sich die axiale Länge von Gewindehülse 10 plus Gewindespindel 25 und folglich der Streckstangen 3. Dadurch wird auf die Druckfeder 42 ein Druck in Richtung des Kopplungselements 4 ausgeübt, wodurch mittels der Befestigungseinrichtung 43 eine Zugkraft auf den Penis ausgeübt wird.

Diese Zugkraft kann aufgrund konstruktiven Ausgestaltung der Streckstangen 3 fein dosiert werden. Zudem ist die erfindungsgemäße Vorrichtung aufgrund von zwei teleskopischen Verstelleinheiten in Form einer Gewindehülse 20 und einer Gewindespindel 25 in Kombination mit einer Gelenkhülse 10 in einem großen Längenbereich einsetzbar, um den verschiedenen Anforderungen gerecht werden zu können. Damit kann mit einer Vorrichtung unterschiedlichen Penisgrößen Rechnung getragen werden.

Die axiale Länge der Gewindehülsen 20 und der Gewindespindel 25 kann beliebig gewählt werden. Somit können für eine Vorrichtung 1 kurze und auch längere Gewindehülsen 20 sowie kurze und auch längere Gewindespindel 25 eingesetzt werden.

### Bezugszeichenliste

• 1 Vorrichtung
2 Stützring
3 längenverstellbare Streckstange
4 Kopplungselement
5 hohlzylindrisches Aufnahmeteil
6 federnde Wange
7 Deckel
8 Verbindungselement
9 Lagerbuchse
10 Gelenkhülse
11 Gelenk
12 Gelenkaufnahme
13 Backe
14 Ausnehmung
15 Gelenkstück
16 Mittelteil
17 halbkugelförmige Ausbuchtung
18 Achse
19 Innenbohrung mit Gewinde der Gelenkhülse 10
20 hohlzylindrische Gewindehülse
21 Außengewinde der Gewindehülse 20
22 Innenbohrung Gewindehülse 20
23 Gewinde Innenbohrung 22
24 Anschlagflansch
25 Gewindespindel
26 Zylinderstift Gewindespindel 25
27 Außengewinde Gewindespindel 25
28 zylindrischer Steckstift Gewindespindel 25
29 Anschlagflansch Gewindespindel 25
30 Rastspitze
31 Einschnitt in Rastspitze 30
32 Rastzunge
33 Rastnase
34 Federhülse
35 Innenbohrung Federhülse 34
36 Absatz in Innenbohrung 35
37 Bohrung
38 Deckel
39 holzylindrischer Fortsatz Deckel 38
40 Rastnase
41 Einschnitt in holzylindrischen Fortsatz 39
42 Druckfeder
43 Befestigungseinrichtung
44 flexible Manschette
45 Vakuumpumpe
^{•} 46 Hohlkörper
47 Lagerzapfen

## Patentansprüche

1. Vorrichtung (1) zur dauerhaften Extension eines Penis, die einen Stützring (2) und mindestens zwei an dem Stützring (2) gelenkig befestigte, mit Hilfe einer Feder (42) axial federnd gelagerte und längenverstellbare Streckstangen (3) umfasst, wobei die Streckstangen (3)
eine Gelenkhülse (10), die an einem Ende gelenkig mit dem Stützring (2) verbunden ist,
eine Federhülse (34), in der die Feder (42) angeordnet ist und die an einem Ende mit einem Kopplungselement (4), das geeignet ist, eine Befestigungseinrichtung (43) für einen Penis mit den Streckstangen (3) zu verbinden und eine Zugkraft auf den Penis auszuüben, verbunden ist, und
mindestens eine zwischen Federhülse (34) und Gelenkhülse (10) angeordnete stangenförmige teleskopischen Verstelleinheit umfassen,
**dadurch gekennzeichnet, dass**
die Gelenkhülse (10) eine Innenbohrung (19) aufweist, in deren Wandung ein Innengewinde (19) ausgebildet ist,
zwischen Federhülse (34) und Gelenkhülse (10) ein erste (20) und eine zweite (25) teleskopische Verstelleinheit angeordnet sind,
die erste Verstelleinheit eine Gewindehülse (20) darstellt, die ein Außengewinde (21), das in das Innengewinde (19) der Gelenkhülse (10) eingeschraubt ist, und eine Innenbohrung (22), in deren Wandung ein Innengewinde (23) ausgebildet ist, besitzt,
die zweite Verstelleinheit eine Gewindespindel (25) darstellt, die zur Gewindehülse (20) hin mit einem Außengewinde (27), das in das Innengewinde (23) der Gewindehülse (20) eingeschraubt ist, und zur Federhülse (34) hin mit einem in die Federhülse (34) ragenden Steckstift (28) ausgestattet ist, und
die Feder (42) in der Federhülse (34) zwischen Steckstift (28) und Kopplungselement (4) angeordnet ist.

2. Vorrichtung nach Anspruch 1
**dadurch gekennzeichnet, dass**
zwischen dem Außengewinde (27) und dem Steckstift (28) der Gewindespindel (25) ein radial außen umlaufender Anschlagflansch (29) ausgebildet ist und/oder die Gewindehülse (20) an ihrem distalen Ende einen radial außen umlaufenden Anschlagflansch (24) besitzt.

3. Vorrichtung nach einem der vorhergehenden Ansprüche
**dadurch gekennzeichnet, dass**
eines, zwei, drei, vier oder fünf der folgenden Merkmale verwirklicht sind:
a. die axiale Länge der Innenbohrung (19) der Gelenkhülse (10) macht mindestens 80 % der axialen Länge der Gelenkhülse (10) aus,
b. die axiale Länge der Innenbohrung (22) der Gewindehülse (20) macht mindestens 80 % der axialen Länge der Gewindehülse (20) aus,
c. die axiale Länge, die für die Ausbildung des Außengewindes (21) der Gewindehülse (20) zur Verfügung steht, beträgt mindestens 80 % der gesamten axialen Länge der Gewindehülse (20).
d. die axiale Länge der Mantelfläche der Gewindehülse (20), die für die Ausbildung des Außengewindes (21) zur Verfügung steht, macht mindestens 80 % der gesamten axialen Länge des Innenbohrung (22) der Gewindehülse (10) aus,
e. die axiale Länge, die für die Ausbildung des Außengewindes (27) der Gewindespindel (25) zur Verfügung steht, beträgt mindestens 80 % der axialen Länge des Innenbohrung (19) der Gewindehülse (20),

4. Vorrichtung nach einem der vorhergehenden Ansprüche
**dadurch gekennzeichnet, dass**
die Federhülse (34) zum Kopplungselement (4) hin durch einen einrastbaren Deckel (38) verschlossen ist und die Feder (42) zwischen Deckel (38) und Steckstift (38) angeordnet ist.

5. Vorrichtung nach einem der vorhergehenden Ansprüche
**dadurch gekennzeichnet, dass**
die Streckstangen (3) an dem Stützring (2) derart befestigt sind, dass der Stützring (2) pro Streckstange (3) zwei parallele Backen (12) besitzt, die jeweils eine nach innen offene Ausnehmung (14) besitzen,
dass die Gelenkhülsen (10) ein Gelenkstück (15), das ein ringscheibenförmiges Mittelteil (16) aufweist, an dem beidseitig jeweils eine halbkugelförmige Ausbuchtung (17) ausgebildet sind, besitzen, die Dicke des ringscheibenfömigen Mittelteils (16) geringer als der Abstand der Backen (12) und
die Ausbuchtungen (17) in die gegenüberliegende Ausnehmung (14) eingreifen.

6. Vorrichtung nach einem der vorhergehenden Ansprüche
**dadurch gekennzeichnet, dass**
das Kopplungslement (4) mit einer Befestigungseinrichtung (43) für einen Penis verbunden ist.

7. Vorrichtung nach einem der Ansprüche 1 bis 6
**dadurch gekennzeichnet, dass**
das Kopplungslement (4) eine Befestigungseinrichtung für einen Penis darstellt.

8. Vorrichtung nach einem der vorhergehenden Ansprüche
**dadurch gekennzeichnet, dass**
die Feder (42) eine Druckfeder, ein Stäbchen aus einem elastischen Material oder ein Röhrchen aus einem elastischen Material darstellt .

9. Vorrichtung nach Anspruch 8
**dadurch gekennzeichnet, dass**
es sich bei dem elastischen Material um Silicon handelt.

## Claims

1. An apparatus (1) for permanently extending a penis, which comprises a support ring (2) and at least two extension rods (3) which are articulatedly fastened to the support ring (2) and are axially resiliently mounted by means of a spring (42) and are longitudinally adjustable, wherein the extension rods (3) comprise
a joint sleeve (10) which is articulatedly connected at one end to the support ring (2),
a spring sleeve (34) in which the spring (42) is arranged and which is connected at one end to a coupling element (4) which is suitable for connecting a fastening device (43) for a penis to the extension rods (3) and for exerting a tensile force on the penis, and
at least one rod-shaped telescopic adjustment unit arranged between the spring sleeve (34) and the joint sleeve (10),
**characterized in that**
the joint sleeve (10) comprises an internal bore (19) in the wall of which an internal thread (19) is formed,
a first (20) and a second (25) telescopic adjustment unit are arranged between the spring sleeve (34) and the joint sleeve (10),
the first adjustment unit provides a threaded sleeve (20) which has an external thread (21) which is screwed into the internal thread (19) of the joint sleeve (10) and an internal bore (22) in the wall of which an internal thread (23) is formed,
the second adjustment unit provides a threaded spindle (25), which is provided towards the threaded sleeve (20) with an external thread (27), which is screwed into the internal thread (23) of the threaded sleeve (20), and towards the spring sleeve (34) with a plug pin (28) projecting into the spring sleeve (34), and
the spring (42) is arranged in the spring sleeve (34) between the plug pin (28) and the coupling element (4).

2. The apparatus according to claim 1,
**characterized in that**
a radially outer circumferential stop flange (29) is formed between the external thread (27) and the plug pin (28) of the threaded spindle (25) and/or the threaded sleeve (20) has a radially outer circumferential stop flange (24) at its distal end.

3. The apparatus according to one of the preceding claims,
**characterized in that**
one, two, three, four or five of the following features are realized:
a. the axial length of the internal bore (19) of the joint sleeve (10) makes up at least 80% of the axial length of the joint sleeve (10),
b. the axial length of the internal bore (22) of the threaded sleeve (20) makes up at least 80% of the axial length of the threaded sleeve (20),
c. the axial length available for forming the external thread (21) of the threaded sleeve (20) is at least 80% of the total axial length of the threaded sleeve (20).
d. the axial length of the outer surface of the threaded sleeve (20), which is available for the formation of the external thread (21), makes up at least 80% of the total axial length of the internal bore (22) of the threaded sleeve (10),
e. the axial length available for forming the external thread (27) of the threaded spindle (25) is at least 80% of the axial length of the internal bore (19) of the threaded sleeve (20),

4. The apparatus according to one of the preceding claims,
**characterized in that**
the spring sleeve (34) is closed towards the coupling element (4) by a latchable cover (38) and the spring (42) is arranged between the cover (38) and the plug pin (38).

5. The apparatus according to one of the preceding claims,
**characterized in that**
the extension rods (3) are fastened to the support ring (2) in such a way
that the support ring (2) has two parallel jaws (12) per extension rod (3), each of which has an inwardly open recess (14),
**in that** the joint sleeves (10) have a joint piece (15), which has an annular disk-shaped central part (16), on which a hemispherical bulge (17) is formed on both sides,
the thickness of the annular disk-shaped central part (16) is less than the distance between the jaws (12) and
the bulges (17) engage in the opposite recess (14).

6. The apparatus according to one of the preceding claims,
**characterized in that**
the coupling element (4) is connected to a fastening device (43) for a penis.

7. The apparatus according to one of claims 1 to 6,
**characterized in that**
the coupling element (4) is a fastening device for a penis.

8. The apparatus according to one of the preceding claims,
**characterized in that**
the spring (42) is a compression spring, a rod made of an elastic material or a tube made of an elastic material.

9. The apparatus according to claim 8,
**characterized in that**
the elastic material is silicone.

## Revendications

1. Dispositif (1) pour l'extension permanente d'un pénis, qui comprend un anneau de soutien (2) et au moins deux tiges d'extension (3) fixées de manière articulée à l'anneau de soutien (2), montées de manière élastique dans la direction axiale à l'aide d'un ressort (42) et réglables en longueur, les tiges d'extension (3) comprenant :
une douille d'articulation (10) qui est reliée de manière articulée à l'anneau de soutien (2) à une extrémité,
une douille à ressort (34) dans laquelle est disposé le ressort (42) et qui est reliée à une extrémité à un élément d'accouplement (4) qui est conçu pour relier un moyen de fixation (43) pour un pénis aux tiges d'extension (3) et pour exercer une force de traction sur le pénis, et
au moins une unité de réglage télescopique en forme de tige disposée entre la douille à ressort (34) et la douille d'articulation (10),
**caractérisé en ce que**
la douille d'articulation (10) comprend un alésage intérieur (19) dans la paroi duquel est formé un filetage intérieur (19),
une première (20) et une deuxième (25) unité de réglage télescopique sont disposées entre la douille à ressort (34) et la douille d'articulation (10),
la première unité de réglage représente une douille filetée (20) qui possède un filetage extérieur (21) vissé dans le filetage intérieur (19) de la douille d'articulation (10), et un alésage intérieur (22) dans la paroi duquel est formé un filetage intérieur (23),
la deuxième unité de réglage représente une broche filetée (25) équipée, vers la douille filetée (20), d'un filetage extérieur (27) vissé dans le filetage intérieur (23) de la douille filetée (20), et, vers la douille à ressort (34), d'une fiche (28) pénétrant dans la douille à ressort (34), et
le ressort (42) est disposé dans la douille à ressort (34) entre la fiche (28) et l'élément d'accouplement (4).

2. Dispositif selon la revendication 1, **caractérisé**
**en ce qu'**une bride de butée (29) périphérique radialement extérieure est formée entre le filetage extérieur (27) et la fiche (28) de la broche filetée (25) et/ou la douille filetée (20) possède à son extrémité distale une bride de butée (24) périphérique radialement extérieure.

3. Dispositif selon l'une quelconque des revendications précédentes, **caractérisé**
**en ce qu'**une, deux, trois, quatre ou cinq des caractéristiques suivantes sont réalisées :
a. la longueur axiale de l'alésage intérieur (19) de la douille d'articulation (10) représente au moins 80 % de la longueur axiale de la douille d'articulation (10),
b. la longueur axiale de l'alésage intérieur (22) de la douille filetée (20) représente au moins 80 % de la longueur axiale de la douille filetée (20),
c. la longueur axiale qui est disponible pour la formation du filetage extérieur (21) de la douille filetée (20) est d'au moins 80 % de la longueur axiale totale de la douille filetée (20),
d. la longueur axiale de la surface extérieure de la douille filetée (20) qui est disponible pour la formation du filetage extérieur (21) représente au moins 80 % de la longueur axiale totale de l'alésage intérieur (22) de la douille filetée (10),
e. la longueur axiale qui est disponible pour la formation du filetage extérieur (27) de la broche filetée (25) est d'au moins 80 % de la longueur axiale de l'alésage intérieur (19) de la douille filetée (20).

4. Dispositif selon l'une quelconque des revendications précédentes, **caractérisé**
**en ce que** la douille à ressort (34) est fermée par un couvercle encliquetable (38) vers l'élément d'accouplement (4), et **en ce que** le ressort (42) est disposé entre le couvercle (38) et la fiche (38).

5. Dispositif selon l'une quelconque des revendications précédentes, **caractérisé**
**en ce que** les tiges d'extension (3) sont fixées à l'anneau de soutien (2) de telle sorte que l'anneau de soutien (2) possède, pour chaque tige d'extension (3), deux mâchoires parallèles (12) possédant chacune un évidement (14) ouvert vers l'intérieur,
**en ce que** les douilles d'articulation (10) possèdent une pièce d'articulation (15) qui comprend une partie centrale en forme de disque annulaire (16), de chaque côté de laquelle est formé un renflement hémisphérique (17), l'épaisseur de la partie centrale en forme de disque annulaire (16) étant inférieure à la distance entre les mâchoires (12) et les renflements (17) s'engageant chacun dans l'évidement (14) qui lui fait face.

6. Dispositif selon l'une quelconque des revendications précédentes, **caractérisé**
**en ce que** l'élément d'accouplement (4) est relié à un moyen de fixation (43) pour un pénis.

7. Dispositif selon l'une quelconque des revendications 1 à 6, **caractérisé**
**en ce que** l'élément d'accouplement (4) représente un moyen de fixation pour un pénis.

8. Dispositif selon l'une quelconque des revendications précédentes, **caractérisé**
**en ce que** le ressort (42) est un ressort de compression, une baguette en matériau élastique ou un petit tube en matériau élastique.

9. Dispositif selon la revendication 8, **caractérisé**
**en ce que** le matériau élastique est du silicone.
